# EUROPEAN PATENT SPECIFICATION

(11) **EP 0 537 585 B1**
(45) Date of publication and mention of the grant of the patent: **20.08.1997**
(21) Application number: 92116980.1
(22) Date of filing: 05.10.1992
(51) Int. Cl.: C07D 251/52, C07D 251/42, C07D 251/48, C07D 239/42, C07D 239/47, C07D 239/48, C07C 333/04

(54) **Process for preparing sulfonylureas**
Verfahren zur Herstellung von Sulfonylharnstoffen
Procédé pour la préparation de sulfonylurées

(30) Priority: 15.10.1991 US 776138
(43) Date of publication of application: 21.04.1993
(73) Proprietor: E.I. DU PONT DE NEMOURS AND COMPANY, Wilmington Delaware 19898 (US)
(72) Inventor: Campopiano, Onorato, Newark, Delaware 19713 (US); Moon, Marcus P., Wilmington, Delaware 19806 (US)
(74) Representative: Woodman, Derek

(56) References cited:
- EP-A- 0 056 969
- EP-A- 0 135 332
- EP-A- 0 173 312
- EP-A- 0 336 587
- WO-A-91/15478
- GB-A- 2 150 139
- US-A- 5 084 086

## Description

### FIELD OF THE INVENTION

This invention is directed to a base-accelerated cyanate process for the preparation of sulfonylurea herbicides.

### STATE OF THE ART

Sulfonylurea herbicides are an extremely potent class of herbicides discovered relatively recently which generally consist of a sulfonylurea bridge, -SO₂NHCONH-, linking two aromatic or heteroaromatic ring structures. Such herbicides have become commercially important. There is therefore a continuing need to discover new processes for their preparation that offers advantages that add to their commercial desirability.

GB-A 2150139 and WO91/15478 describe processes for the preparation of sulfonylureas which comprise reacting a sulfonyl halide with a cyanate salt and a suitable amine.

### SUMMARY OF THE INVENTION

This invention pertains to a process for the preparation of sulfonylureas of Formula I comprising reacting a sulfonyl chloride of Formula III with a cyanate salt of Formula IV in the presence of heterocyclic amine of Formula V and a base acording to Equation 1 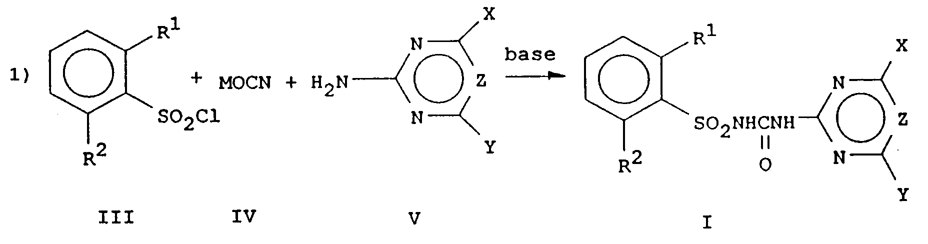 wherein
R¹ is selected from the group CO₂R³ and CHFCH₃;
R² is selected from the group CH₃, CH₂CN and Cl;
R³ is selected from the group C₁-C₃ alkyl;
M is selected from the group Na, K and NH₄;
X is selected from the group CH₃ and N(CH₃)₂;
Y is selected from the group CH₃, OCH₃ and OCH₂CF₃; and
Z is selected from the group CH and N.

The compounds of Formula III may be prepared from intermediates of Formula II 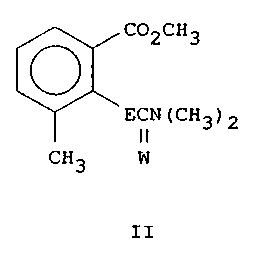 wherein
E is selected from the group O and S; and
W is selected from the group O and S;
   provided that E and W are not simultaneously O or S.

In the above recitations, C₁-C₃ alkyl denotes methyl, ethyl, propyl and isopropyl.

Typical reaction conditions are as follows. The solvent is selected from aprotic solvents with preferred solvents selected from acetonitrile, acetone, dioxane, methylene chloride, tetrahydrofuran and suitable combinations thereof. The most preferred solvent is acetonitrile. Temperatures are 0 to 50°C. Preferred temperatures are 20 to 30°C. Reaction pressures are 1 to 5 atmospheres (1.01 - 5.05 x 10⁵ Pa). The preferred pressure is 1 atmosphere (1.01 x 10⁵ Pa). Reaction times are 0.1 to 24 hours, preferably 1 to 3 hours. The bases are selected from pyridine, picoline, lutidine, sodium bicarbonate, sodium carbonate, sodium acetate and combinations thereof. The preferred base is pyridine. Preferred mole ratio of the compound of Formula III to base is 1:0.5 to 1:2.

Specifically preferred processes for the greatest utility of their products are:
1) The process of Equation 1 wherein R¹ is CO₂R³, R² is CH₃, R³ is CH₃, Z is N, X is N(CH₃)₂ and Y is OCH₂CF₃.
2) The process of Equation 1 wherein R¹ is CO₂R³, R² is Cl, R³ is CH(CH₃)₂, Z is CH and X and Y are CH₃.
3) The process of Equation 1 wherein R¹ is CHFCH₃, R² is Cl, Z is N, X is CH₃ and Y is OCH₃.
4) The process of Equation 1 wherein R¹ is CO₂R³, R² is CH₂CN, R³ is CH₃, Z is CH and X and Y are CH₃.

The sulfonyl chlorides of Formula III are either known or can be prepared by various methods known to one skilled in the art. For example, the compounds of Formula III can be prepared by i) oxidative chlorination of thioethers such as taught in Recl. Trav. Chim. Pays-Bas 101, 91 (1982), ii) diazotization of aromatic amines with sodium nitrite in hydrochloric acid, followed by reaction of the resulting diazonium salt with sulfur dioxide and cuprous chloride as described in J. Org. Chem., 25, 1824 (1960), iii) heteroatom-facilitated lithiation, followed by sulfonation as taught in EP-A-73,562 and reviewed in Org. Reactions, 26 1 (1979).

In addition, sulfonyl chlorides of Formula III can be prepared from substituted phenols via a Newman rearrangement (See J. Org. Chem., 31, 3980 (1966) as illustrated below in Scheme 1.

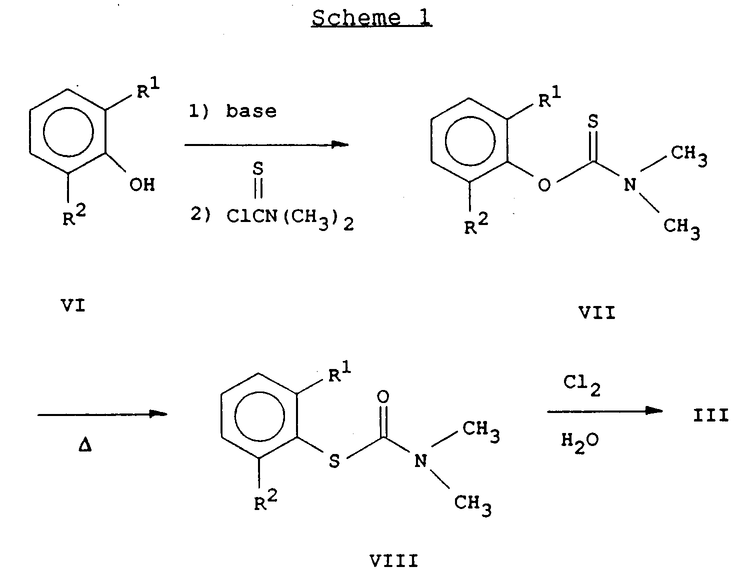

The phenol VI is reacted with a base such as sodium or potassium hydroxide or sodium hydride in an appropriate solvent such as a lower alcohol or inert aprotic solvent followed by treatment with a dialkyl thiocarbamoyl chloride to form a thiocarbamate of Formula VII (See Syn. Comm., 17, 1761 (1987)). The thiocarbamate VII, which need not be isolated, is heated at temperatures of 100-250°C, neat or dissolved in an appropriate solvent, such as o-dichlorobenzene, to effect rearrangement to VIII. Compounds of Formula VIII are readily converted to the sulfonyl chloride derivative III by reaction with chlorine gas in the presence of water or alternatively by action of aqueous sodium hypochlorite with acid.

The amines of Formula V are also either known or can be prepared by methods known in the art. For example, see "The Chemistry of Heterocyclic Compounds," Vol. 13 and 16, Interscience Publishers, Inc., New York and "The Chemistry of the Amino Group," Edited by S. Patai, Interscience Publishers, Inc., 1968, pages 37-77.

The process of this invention is best carried out in a solvent such as acetonitrile, dioxane, methylene chloride, tetrahydrofuran, or combinations thereof. The preferred solvent for greater reactivity and ease of handling is acetonitrile, when an alkali metal cyanate is used.

The cyanate salts IV are ammonium, sodium or potassium. Preferred for greater reactivity and availability are sodium and potassium cyanate. The reaction temperature is in the range of 0 to about 50°C, with ambient temperature preferred for ease of operation. The reaction pressure is 1 to 5 atmospheres (1.01 - 5.05 x 10⁵ Pa), with 1 atmosphere preferred (1.01 x 10⁵ Pa).

The reaction time is determined by the reactivity of the starting materials. In some cases, the reaction is complete after a few minutes while in other cases a reaction time up to about 24 hours is advantageous. The preferred reaction time is normally in the range of 1 to 3 hours.

The ratio of reactants is determined by their reactivity. The sulfonyl chloride (III) and heterocyclic amine (V) are normally used in about a 1:1 to 1.2:1 mole ratio. The cyanate salt is normally used in excess. Mole ratios of sulfonyl chloride to cyanate salt can be 1:1 to 1:10 with 1:2 preferred for reasons of ease of operation and efficiency.

The mole ratio of sulfonyl chloride to base is generally 1:0.1 to 1:3, with 1:0.5 to 1:2 preferred and 1:1 to 1:2 being most preferred. Amounts of base less than the minimum indicated will produce Compound I but the yield will be reduced and/or longer reaction times will result. The bases are selected from pyridine, picoline, lutidine, sodium bicarbonate, sodium carbonate, sodium acetate and combinations thereof, with pyridine as the preferred base. The base accelerates the reaction and significantly improves the yield and ease of isolation of the final product.

The resulting sulfonylurea can be isolated by several methods depending on the solvent, the solubility of the product and the presence of by-products. In some cases, the reaction can be diluted with water, acidified and filtered to give essentially pure sulfonylurea. In other cases, the product can be dissolved in dilute base, filtered, and the filtrate acidified to reprecipitate the product. In still other cases, the product is recrystallized to yield a purified product.

The process of this invention is further illustrated by the following examples.

### EXAMPLE 1

### Preparation of methyl 2-(chlorosulfonyl)-3-methyl benzoate

A mixture of 12 g of KOH pellets, 150 mL of methanol and 24.9 g of methyl 2-hydroxy-3-methylbenzoate (I) was stirred at room temperature for 0.5 hours. The mixture was then cooled to 5°C and 19 g of dimethylthiocarbamoyl chloride was added in one portion. An exotherm of 5°C was observed. The resulting mixture was stirred for 2 hours and then added to 300 mL of ice water. After filtering, the crystalline product was washed with water and oven dried to yield 30-35 g of methyl 2-[(dimethylamino)thioxomethoxy]-3-methylbenzoate II, (E is O, W is S) in 80-92% yield, mp 94-96°C.
¹H NMR (300 MHz, CDCl₃) δ 2.30 (s, 3H), 3.50 (d, 6H), 3.85 (s, 3H), 7.25 (m, 1H), 7.45 (d, 1H), 7.85 (d, 1H).
Mass Spec m/e: 253 (m⁺), 220, 194, 149, 121, 88 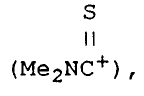 72.

In a reaction flask equipped for stirring was charged 15 g of the product above and 100 mL o-dichlorobenzene. It was heated to reflux under nitrogen atmosphere for 18 hours overnight to effect the Newman-Kwart rearrangement to II ( E is S, W is O). The rearrangement was complete as determined by GC analysis. The structure of the product II (E is S, W is O) was identified by GC/MS, m/e: 253 (m⁺), 220, 194, 149, 121, 77, 72 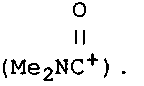 The absence of 88 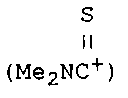 indicates a complete rearrangement. The same reaction can be carried out without solvent by heating the solids directly in a silicone oil bath at 220°C for 6 hours to effect the Newman-Kwart rearrangement to II (E is S, W is O). The rearrangement can be monitored by GC. The rearranged product was characterized by ¹H NMR (300 MHz, CDCl₃) δ 2.50 (s, 3H), 3.10 (br.d, 6H), 3.90 (s, 3H), 7.35 (m, 1H), 7.42 (m, 1H), 7.60 (m, 1H) and by GC/MS, m/e: 253 (m+), 220, 194, 149, 121, 77, 72

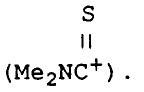

The above rearranged product in o-dichlorobenzene was added 100 mL H₂O and chlorinated at 20°C. A total of 20.7 g chlorine was charged over one hour. After stirring for another hour, the organic layer was rotovapped under vacuum to yield an orange liquid which crystallized upon cooling. It was worked up by dissolving in 100 mL methanol and then chilled in dry ice-acetone bath to crystallize. Filtration and drying afforded 9.7 g of white crystals (65%) yield which was identified to be methyl 2-(chlorosulfonyl)-3-methylbenzoate, mp 115-116°C.
¹H NMR (CDCl₃) δ 2.80 (s, 3H), 3.90 (s, 3H), 7.35 (d, 1H), 7.45 (d, 1H), 7.60 (t, 1H). m/e = 248.

### EXAMPLE 2

### Preparation of Methyl 2-nitro-3-methylbenzoate

Thionylchloride (253 mL) was added dropwise to 3 l methanol at 0-10°C. Toward end of the addition, the temperature was allowed to rise to 20°C. Solid 2-nitro-3-methylbenzoic acid (500 g) was charged in one portion and the reaction mixture was heated to reflux overnight. It was distilled until pot reached 73°C. After cooling to 60°C, 685 mL water was introduced dropwise and the reaction mixture was cooled to 20°C. Upon adding 26 mL water and 125 mL 80% NaOH, the pH was adjusted to 7. The product was collected by filtration and washed with 3 X 100 mL water and air dried overnight. Yield of methyl 2-nitro-3-methylbenzoate was 503.5 g (93.5%), mp 72°C.
¹H NMR (300 MHz, CDCl₃) δ 2.38 (s, 3H), 3.90 (s, 3H), 7.50 (m, 2H), 7.88 (m, 1H).

### EXAMPLE 3

### Preparation of Methyl 2-benzylthio-3-methylbenzoate

In a 5 l pot was charged 483 g of methyl 2-nitro-3-methylbenzoate, 319 g benzyl mercaptan and 100 mL dimethyl formamide. Upon cooling to 0°C, a solution of 321 g potassium tert-butoxide in 1500 mL dimethyl formamide was added dropwise over 2.5 hours at 0-5°C. The reaction mixture was stirred at 0°C for 1.5 hours and then at room temperature overnight. The reaction was quenched by addition of 1 l water and worked up by extraction with 2 l methylene chloride. Upon washing with water (3 x 1 l), the methylene chloride layer was dried over MgSO₄, filtered and rotovapped to give 517 g of crude methyl 2-benzylthio-3-methylbenzoate (77% yield) as a liquid.
¹H NMR (300 MHz, CDCl₃) δ 2.30 (s, 3H), 3.90 (s, 3H), 3.95 (s, 2H), 7.10-7.40 (m, 8H).

### EXAMPLE 4

### Preparation of Methyl 2-(chlorosulfonyl)-3-methyl benzoate

Chlorine gas (542 g) was fed into a solution of methyl 2-benzylthio-3-methylbenzoate (417 g), methylene chloride (2 l) and water (1 l) over 5.5 hours at 20-25°C. Upon complete addition, the reaction mixture was stirred at room temperature for another hour and then allowed to stand overnight. After phase cut, the organic layer was solvent exchanged into hexanes on rotovap. Upon cooling, the solids were filtered and washed with hexanes. After air drying, 179 g of methyl 2-(chlorosulfonyl)-3-methylbenzoate were obtained, mp 114-116°C.
¹H NMR (300 MHz, CDCl₃) δ 2.80 (s, 3H), 3.90 (s, 3H), 7.35 (d, 1H), 7.45 (d, 1H), 7.60 (t, 1H).

### EXAMPLE 5

### Preparation of Methyl 2-[[[[[4-(dimethylamino)-6-(2,2,2-trifluoroethoxy)-1,3,5-triazin-2-yl]amino]carbonyl]amino]sulfonyl]-3-methylbenzoate

To a stirred mixture of 26.3 g (0.41 moles) of sodium cyanate and 33 mL of pyridine (0.4 moles) in 670 mL of acetonitrile was added 47.4 g (0.2 moles) of 4-(dimethylamino)-6-(2,2,2-trifluoroethoxy-1,3,5-triazin-2-amine, followed by 60.31 g (0.24 moles) of methyl-2-(chlorosulfonyl)-3-methyl benzoate in one portion. Upon addition of the sulfonyl chloride an endotherm occurred. The reaction flask was immersed in a water bath, the reaction mixture stirred for 2.3 hours and then poured into about 1.5 L of ice water while stirring. Concentrated HCl (36%) was added in portions to bring the pH of the mixture from an initial 6 to 1. The solids were collected by filtration and the filter cake washed repeatedly with 20 mL portions of methanol. The solid was dried over 3 days in a vacuum oven at 40°C to yield 65.18 g of an off white solid (65%), mp 104-110°C. Melting points of this material have been seen to be widely variable depending on mode of synthesis, crystallization solvent, and degree of hydration. A more reliable criterion of purity is ¹H-NMR and LC.
¹H NMR (300 MHz, CDCl₃) δ 12.40 (s, 1H, N-H), 7.5 (m, 1H, aromatic), 7.4-7.2 (m, 2H, aromatic), 7.10 (s, 1H, N-H), 4.74 (q, 2H, CH₂CF₃), 3.9 (s, 3H, OCH₃), 3.21 (s, 6H, N(CH₃)₂, 2.95 (s, 3H, C-CH₃).

The purity of the above product was determined by LC to be 97.2%.

A sample of the above sulfonylurea (1 g) was slurried in 10 ml methanol at room temperature. Upon adding 0.5 g of 25% NaOMe solution, a clear solution formed which crystallized again after a few minutes. The crystals were filtered and dried in vacuum oven at 40°C overnight to give 0.9 g of the sodium salt of sulfonylurea. The purity of the salt was determined by LC to be 99.6%.

The process of the present invention produces compounds useful as herbicides. Compounds of Formula I are herbicidal. Compounds of Formula I can be isolated in two crystal forms. Thus the process of this invention includes the preparation of either or both crystal forms of compounds of Formula I.

## Claims

1. A process for preparing sulfonylurea compounds of the Formula I 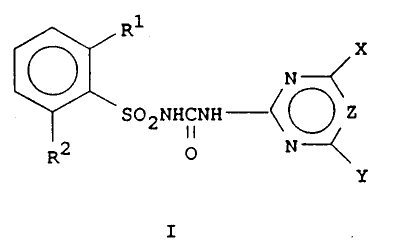 said process comprising reacting a sulfonyl chloride of Formula III 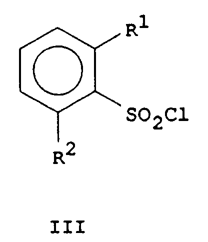 with a cyanate salt of the Formula IV
MOCN IV
in the presence of a heterocyclic amine of the Formula V 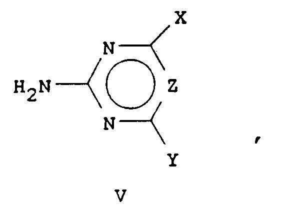 0.1 to 3 mols of base per mol of sulfonyl chloride of Formula III, the base being selected from pyridine, picoline, lutidine, sodium bicarbonate, sodium carbonate, sodium acetate and combinations thereof and an aprotic solvent at a temperature of from 0 to 50°C and a pressure of from 1 to 5 atmospheres (1.01 - 5.05 x 10⁵ Pa); wherein
R¹ is selected from the group CO₂R³ and CHFCH₃;
R² is selected from the group CH₃, CH₂CN and Cl;
R³ is selected from the group C₁-C₃ alkyl;
M is selected from the group Na, K and NH₄;
X is selected from the group CH₃ and N(CH₃)₂;
Y is selected from the group CH₃, OCH₃ and OCH₂CF₃; and
Z is selected from the group CH and N.

2. The process of Claim 1 wherein the amount of base is 0.5 to 2 mols per mol of sulfonyl chloride of Formula III, the temperature is 20 to 30°C, the pressure is one atmosphere (1.01 x 10⁵ Pa) and the solvent is selected from acetonitrile, dioxane, methylene chloride, tetrahydrofuran and combinations thereof.

3. The process of Claim 2 wherein
R¹ is CO₂R³;
R² is CH₃;
R³ is CH₃;
Z is N;
X is N(CH₃)₂; and
Y is OCH₂CF₃.

4. The process of Claim 2 wherein
R¹ is CO₂R³;
R² is Cl;
R³ is CH(CH₃)₂;
Z is CH;
X is CH₃; and
Y is CH₃.

5. The process of Claim 2 wherein
R¹ is CHFCH₃;
R² is Cl;
Z is N;
X is CH₃; and
Y is OCH₃.

6. The process of Claim 2 wherein
R¹ is CO₂R³;
R² is CH₂CN;
R³ is CH₃;
Z is CH; and
X and Y are CH₃.

7. The process of Claim 3 wherein
the cyanate salt of Formula IV is selected from sodium cyanate and potassium cyanate;
the solvent is acetonitrile; and
the base is pyridine.

8. The process of Claim 2 wherein the base is pyridine.

9. The process of Claim 8 wherein the cyanate salt of Formula IV is selected from sodium cyanate and potassium cyanate.

## Patentansprüche

1. Verfahren zur Herstellung von Sulfonylharnstoff-Verbindungen der Formel I 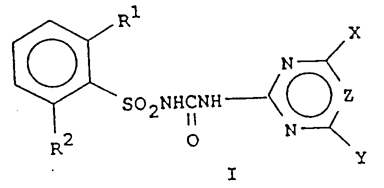 wobei das Verfahren die Umsetzung eines Sulfonylchlorides der Formel III 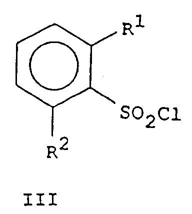 mit einem Cyanatsalz der Formel IV
MOCN IV
in Gegenwart von einem heterocyclischen Amin der Formel V 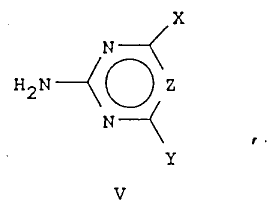 0,1 bis 3 mol Base pro mol Sulfonylchlorid der Formel III, wobei die Base ausgewählt ist aus Pyridin, Picolin, Lutidin, Natriumbicarbonat, Natriumcarbonat, Natriumacetat und aus Kombinationen davon, und einem aprotischen Lösungsmittel bei einer Temperatur von 0 bis 50 °C und bei einem Druck von 1 bis 5 Atmosphären (1,01 - 5,05x10⁵ Pa) umfaßt, worin
R¹ ausgewählt ist aus der Gruppe CO₂R³ und CHFCH₃;
R² ausgewählt ist aus der Gruppe CH₃, CH₂CN und Cl;
R³ ausgewählt ist aus der Gruppe C₁-C₃-Alkyl;
M ausgewählt ist aus der Gruppe Na, K und NH₄;
X ausgewählt ist aus der Gruppe CH₃ und N(CH₃)₂;
Y ausgewählt ist aus der Gruppe CH₃, OCH₃ und OCH₂CF₃; und
Z ausgewählt ist aus der Gruppe CH und N.

2. Verfahren nach Anspruch 1, bei dem die Menge der Base 0,5 bis 2 mol pro mol Sulfonylchlorid der Formel III beträgt, die Temperatur 20 bis 30 °C beträgt, der Druck 1 Atmosphäre (1,01x10⁵ Pa) beträgt und das Lösungsmittel aus Acetonitril, Dioxan, Methylenchlorid, Tetrahydrofuran und aus Kombinationen davon ausgewählt wird.

3. Verfahren nach Anspruch 2, bei dem
R¹ für CO₂R³ steht;
R² für CH₃ steht;
R³ für CH₃ steht
Z für N steht;
X für N(CH₃)₂ steht; und
Y für OCH₂CF₃ steht.

4. Verfahren nach Anspruch 2, bei dem
R¹ für CO₂R³ steht;
R² für Cl steht;
R³ für CH(CH₃)₂ steht;
Z für CH steht;
X für CH₃ steht; und
Y für CH₃ steht.

5. Verfahren nach Anspruch 2, bei dem
R¹ für CHFCH₃ steht;
R² für Cl steht;
Z für N steht;
X für CH₃ steht; und
Y für OCH₃ steht.

6. Verfahren nach Anspruch 2, bei dem
R¹ für CO₂R³ steht;
R² für CH₂CN steht;
R³ für CH₃ steht;
Z für CH steht; und
X und Y für CH₃ stehen.

7. Verfahren nach Anspruch 3, bei dem das Cyanatsalz der Formel IV ausgewählt wird aus Natriumcyanat und Kaliumcynat, das Lösungsmittel Acetonitril ist und die Base Pyridin ist.

8. Verfahren nach Anspruch 2, bei dem die Base Pyridin ist.

9. Verfahren nah Anspruch 8, bei dem das Cyanatsalz der Formal IV ausgewählt ist aus Natriumcyanat und Kaliumcyanat.

## Revendications

1. Procédé de préparation de composés sulfonylurées répondant à la formule I 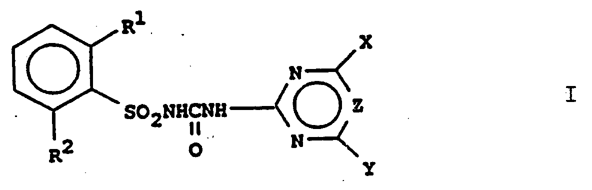 ledit procédé comprenant la mise en réaction d'un chlorure de sulfonyle de formule III 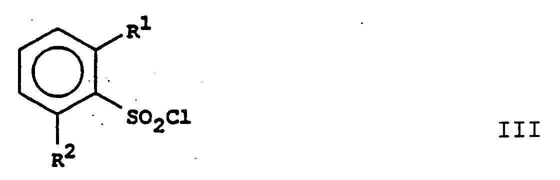 avec un sel cyanate de formule IV
MOCN IV
en présence d'une amine hétérocyclique de formule V 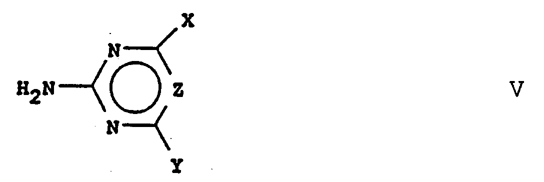 de 0,1 à 3 modes de base par mode de chlorure de sulfonyle de formule III, la base étant choisie parmi la pyridine, la picoline, la lutidine, le bicarbonate de sodium, le carbonate de sodium, l'acétate de sodium et des combinaisons de ceux-ci, et d'un solvant aprotique, à une température de 0 à 50°C et à une pression de 1 à 5 atmosphères (1,01 à 5,05 x 10⁵ Pa),
où
R¹ est choisi dans d'ensemble constitué par CO₂R³ et CHFCH₃ ;
R² est choisi dans l'ensemble constitué par CH₃, CH₂CN et Cl ;
R³ est choisi dans d'ensemble constitué par des groupes alkyles en C₁-C₃ ;
M est choisi dans l'ensemble constitué par Na, K et NH₄ ;
X est choisi dans d'ensemble constitué par CH₃ et N(CH₃)₂ ;
Y est choisi dans l'ensemble constitué par CH₃, OCH₃ et OCH₂CF₃ ; et
Z est choisi dans l'ensemble constitué par CH et N.

2. Procédé selon la revendication 1, dans lequel la quantité de base est de 0,5 à 2 moles par mole de chlorure de sulfonyle de formule III, la température est de 20 à 30°C, la pression est de 1 atmosphère (1,01 x 10⁵ Pa), et le solvant est choisi parmi l'acétonitrile, le dioxanne, le chlorure de méthylène, le tétrahydrofuranne, et les combinaisons de ceux-ci.

3. Procédé selon la revendication 2, dans lequel R¹ est CO₂R³, R² est CH₃, R³ est CH₃, Z est N, X est N(CH₃)₂ et Y est OCH₂CF₃.

4. Procédé selon la revendication 2, dans lequel R¹ est CO₂R³, R² est Cl, R³ est CH(CH₃)₂, Z est CH, X est CH₃ et Y est CH₃.

5. Procédé selon la revendication 2, dans lequel R¹ est CHFCH₃, R² est Cl, Z est N, X est CH₃ et Y est OCH₃.

6. Procédé selon la revendication 2, dans lequel R¹ est CO₂R³, R² est CH₂CN, R³ est CH₃, Z est CH, et X et Y sont CH₃.

7. Procédé selon la revendication 3, dans lequel le sel cyanate de formule IV est choisi parmi le cyanate de sodium et le cyanate de potassium ; le sodium est l'acétonitrile ; et la base est la pyridine.

8. Procédé selon la revendication 2, dans lequel la base est la pyridine.

9. Procédé selon la revendication 8, dans lequel le sel cyanate de formule IV est choisi parmi le cyanate de sodium et le cyanate de potassium.
